# EUROPEAN PATENT SPECIFICATION

(11) **EP 4 755 433 B1**
(45) Date of publication and mention of the grant of the patent: **18.11.2026**
(21) Application number: 24834582.9
(22) Date of filing: 22.08.2024
(51) Int. Cl.: A61M 16/16, A61M 16/10, A61M 16/08, B29C 65/08, B29C 65/00, B29C 65/48

(54) **HUMIDIFIER WITH CHEMICAL & ADHESIVE FREE TUBE CONNECTOR FOR MEDICAL EQUIPMENT**
BEFEUCHTER MIT CHEMIKALIEN- UND KLEBSTOFFFREIEM ROHRANSCHLUSS FÜR MEDIZINISCHE EINRICHTUNG
HUMIDIFICATEUR AYANT UN RACCORD DE TUBE LIBRE DE PRODUITS CHIMIQUES ET D'ADHÉSIFS POUR ÉQUIPEMENT RESPIRATOIRE

(30) Priority: 16.08.2024 CN 202411130829
(43) Date of publication of application: 10.06.2026
(73) Proprietor: Vincent Medical (Dong Guan) Technology Co., Ltd, Dongguan, Guangdong 523808 (CN); Vincent Medical Admired Co., Ltd., Dongguan, Guangdong 523000 (CN); Vincent Medical (Dong Guan) Manufacturing Co., Ltd., Dongguan, Guangdong 523730 (CN)
(72) Inventor: NG, Kam Man, Tangxia Town, Dongguan, Guangdong 523730 (CN); CHOI, Cheung Tai Raymond, Tangxia Town, Dongguan, Guangdong 523730 (CN); LI, Siu Tung, Songshan Lake Zone, Dongguan, Guangdong 523808 (CN); FENG, Zhiwei, Songshan Lake Zone, Dongguan, Guangdong 523808 (CN)
(74) Representative: KIPA AB
(86) International application number: PCT/CN2024/114039
(87) International publication number: WO 2026/036428

(56) References cited:
- WO-A1-2020/044291
- CN-A- 114 072 192
- CN-U- 204 421 298
- CN-U- 210 612 661
- CN-U- 212 593 420
- CN-U- 212 914 153
- CN-U- 213 158 745
- CN-U- 214 912 289
- CN-U- 214 912 289
- CN-U- 214 970 794
- CN-U- 214 970 794
- CN-U- 215 690 825
- CN-U- 215 690 825
- CN-U- 217 187 326
- CN-U- 221 384 794
- US-A1- 2017 252 531
- US-A1- 2018 296 791
- US-A1- 2022 016 383

## Description

### TECHNICAL FIELD

The present disclosure relates to the technical field of respiratory equipment, and in particular, to a humidifier with a glue-free chemical solvent-free dropper bottle tube connector and respiratory equipment.

### BACKGROUND

At present, an assembly process for a humidifier and an infusion tube generally adopts glue for curing and connecting. To ensure stable curing and connecting of the glue, the assembly process for the humidifier and the infusion tube usually needs to be air-dried for a long time or irradiated by using UV light to accelerate curing.

However, after assembly is completed, an existing glue curing process has a problem of volatilization of volatile organic matters, which poses a safety risk for a patient when using the humidifier.

Therefore, a conventional art has defects and disadvantages, which needs to be further improved and developed.

WO 2020/044291 A1 discloses a humidification assembly or kit for use in a respiratory support system for delivering humidified gas to a user or patient. The assembly or kit comprises a fluid reservoir, and a humidification chamber. The fluid reservoir and the humidification chamber are provided in fluid connection with each other, wherein a connection between each of the fluid reservoir and the humidification chamber provides a pre-determined force of retention and/or resistance to disconnection or is a permanent type connection.

CN 204 421 298 U discloses a water tank assembly and a humidifier. The water tank assembly comprises a water supply element, a humidifying pipe, a first connecting pipe, a water tank cover, and a water tank. The humidifying pipe, the first connecting pipe, the water tank cover, and the water tank are all in communication with the water supply element. One end of the first connecting pipe is connected to the humidifying pipe, and the other end is detachably connected to and in communication with the water tank cover. The water tank cover is detachably connected to and in communication with the water tank. The arrangement of the first connecting pipe between the humidifying pipe and the water tank cover, in conjunction with the threaded connection between the water tank cover and the water tank, provides an effective seal that prevents water leakage. This sealing operation is simple to perform, requires no specialized procedures, and ensures high reliability.

CN 214 912 289 U discloses a humidifying device for a breathing machine and a humidifying tank thereof. The device comprises a tank body having an air inlet and an air outlet. A water injection connector is further provided on the tank body, which includes an outer cover and an inner pipe. The inner pipe is in communication with the interior of the tank body, while the outer cover encloses the inner pipe and is connected to the tank body or the inner pipe. The inner wall of the outer cover is provided with internal threads. In use, a second connector on an infusion apparatus is connected to the water injection connector of the humidifying tank. This allows water to be continuously provided to the humidifying tank in a dripping mode, ensuring that the water level within the tank is maintained at a constant level. This design eliminates the need to repeatedly draw and inject distilled water into the humidifying tank with a syringe and to repeatedly open the tank's water injection port. The advantages of this utility model are that the operation is simple and convenient, thereby reducing the risk that the water filling port is left improperly closed. Furthermore, medical staff are not required to frequently monitor the water level in the humidifying tank, which significantly reduces their workload.

CN 215 690 825 U discloses medical auxiliary devices, specifically an intelligent water adding device for a humidifying tank. The device comprises a humidifying tank body equipped with a pressure sensor on its right-bottom side. The base of the tank body features a humidifying rear air inlet end, a standard air inlet end positioned behind it, and a water adding end positioned to its right. The water adding end is fitted with a water adding pipeline assembly, which terminates in an infusion apparatus. A water level line is marked on the upper-right portion of the tank's front face. A protective cap assembly is installed at the humidifying rear air inlet end and consists of a connecting strip, a protective cap cover, an opening and closing auxiliary block, and a protective plug. The overall structure is simple and effectively relieves nurses of the workload associated with manually adding water to the tank.

CN 221 384 794 U discloses a breathing machine humidifying tank convenient for fluid infusion, which comprises a humidifying tank, the top of the humidifying tank is in threaded connection with an auxiliary mechanism, and the auxiliary mechanism comprises a plugging component and a fluid infusion component. The plugging assembly is in threaded connection with the liquid injection pipe through the threaded column, so that the humidifying tank is plugged, meanwhile, the water level sensor of the plugging assembly is inserted into the inner side of the humidifying tank, the storage amount of liquid in the inner side of the humidifying tank can be monitored, and when the water level drops to the state where liquid needs to be supplemented, the alarm is started through the control module to give out early warning, so that the humidifying tank is prevented from being blocked. An operator can conveniently know the consumption condition of the humidifying liquid in time; when liquid supplementing is needed, the inserting pipe can be aligned to the first threaded cylinder on the plugging assembly, the second threaded cylinder is connected to the outer side of the first threaded cylinder in a threaded mode, and therefore the inserting pipe is driven to penetrate through the plugging ring in an inserting mode through screwing to conduct liquid supplementing operation, an operator does not need to hold the liquid supplementing device during liquid supplementing, and liquid supplementing is stable.

CN 210 612 661 U discloses an automatic water replenishing device for an oxygen therapy system. The system comprises an oxygen bottle, a humidifier, and an oxygen inhaler, all connected in fluid communication by an air guide pipe. The humidifier body is transparent and has an upper end that is conical in shape. A liquid replenishing port is formed at the upper end of the humidifier body, where its opening is designed to be flush with the inner wall of the humidifier to facilitate seamless fluid transfer. This port is connected to the automatic water replenishing device. The automatic water replenishing device consists of a flow adjusting device and a liquid supplementing bottle, which are sequentially connected in series with the liquid replenishing port via guide pipes. The liquid supplementing bottle is positioned at a height that is not lower than the liquid replenishing port. This configuration allows the device to automatically deliver sterilized distilled water into the humidifier at a set flow rate, thereby reducing the workload and operational intensity for medical staff.

CN 213 158 745 U discloses a humidifying tank device for a breathing machine, comprising a humidifying tank body and an infusion apparatus. A water adding port is provided on the tank body. Surrounding the bottom end of this port is a permanent magnet. Below the water adding port is a detachably mounted pipe body, whose upper end contains an embedded iron ring that is attracted to the permanent magnet, securing the pipe body in place. The upper end of the pipe body is open, with through holes in its peripheral wall, and its lower end is sealed by an end cover having a central through hole. A float component is mounted within the pipe body, consisting of a connecting rod with a rubber hollow ball fixed at its upper end and a hollow floater body at its lower end. The rubber hollow ball is contained within the pipe body, while the connecting rod's lower end extends through the end cover's through hole. The lower end connector of the infusion apparatus's pipeline comprises a central intubation tube and a peripheral ferrule. The central intubation tube is designed for insertion into the humidifying tank's water adding port, while the peripheral ferrule is designed to fit tightly around the port's periphery, ensuring a secure connection.

### SUMMARY

In view of the foregoing disadvantages in a conventional art, an objective of the present disclosure is to provide a glue-free chemical solvent-free dropper bottle tube connector and respiratory equipment, which aims to solve a problem of volatilization of volatile organic matters in an existing assembly process for a humidifier and an infusion tube in the conventional art. The invention is defined by the appended claims.

A technical solution adopted in the present disclosure for solving the technical problem is as follows: a humidifier with a glue-free chemical solvent-free dropper bottle tube connector, configured for respiratory equipment. The humidifier includes:
an infusion tube;
a humidifier body, where the humidifier body has an upper cover end and a base plate end arranged opposite to each other;
a water inlet, where the water inlet is fixedly provided on the upper cover end, the water inlet is provided with a water inlet hole, and a plurality of locking parts are fixedly arranged on the water inlet;
an infusion tube connector, where the infusion tube connector has an insertion section, a limiting boss, and a hole entrance section arranged in sequence from top to bottom, the hole entrance section is detachably connected to the water inlet hole, and the insertion section is detachably connected to the infusion tube; and
a connector locking fastener, where the connector locking fastener is provided with a perforation hole, a plurality of fixing pieces are fixedly arranged on the connector locking fastener, and the plurality of fixing pieces are configured to cooperate with the locking parts and the infusion tube connector to fix the infusion tube to the water inlet hole.

The infusion tube connector includes an ultrasonic line, where the ultrasonic line is annularly arranged at an end of the limiting boss deviating from the insertion section, and the ultrasonic line is configured to perform ultrasonic welding on the water inlet; and
an inverted fastener, where the inverted fastener is fixedly arranged on the insertion section.

Optionally, a cross section of the ultrasonic line along an axial direction of the infusion tube connector is arranged in a V shape, a triangle, or a cone shape.

Optionally, a diameter of the ultrasonic line is set as 1.2 times-2 times that of the water inlet hole.

Optionally, each of the plurality of locking parts includes a locking boss, and the locking boss is fixedly provided on a side wall of the water inlet.

Optionally, each of the plurality of fixing pieces includes a plurality of rotary fasteners, the plurality of rotary fasteners are fixedly arranged on an inner side wall of the connector locking fastener, and the plurality of rotary fasteners are configured to cooperate with the locking bosses and the infusion tube connector to fix the infusion tube to the water inlet hole.

The infusion tube penetrates out from the perforation hole, the infusion tube is sleeved in the insertion section, the hole entrance section is inserted into the water inlet hole, the limiting boss is propped against a hole edge of the water inlet hole, ultrasonic welding is performed on the water inlet and the infusion tube connector by using the ultrasonic line, the connector locking fastener is sleeved over the water inlet, and the plurality of rotary fasteners are rotated into the locking bosses by rotating the connector locking fastener.

Optionally, each of the plurality of locking parts includes a clamping boss, and the clamping boss is provided on a side wall of the water inlet.

Optionally, each of the plurality of fixing pieces includes a clamping groove, the plurality of clamping grooves are provided in a side wall of the connector locking fastener, the clamping bosses are adapted to the clamping grooves in dimensions, and the plurality of clamping grooves are configured to cooperate with the clamping bosses and the infusion tube connector to fix the infusion tube to the water inlet hole.

The infusion tube penetrates out from the perforation hole, the infusion tube is sleeved in the insertion section, the hole entrance section is inserted into the water inlet hole, the limiting boss is propped against a hole edge of the water inlet hole, ultrasonic welding is performed on the water inlet and the infusion tube connector by using the ultrasonic line, the connector locking fastener is sleeved over the water inlet, and the plurality of clamping grooves are all buckled in the clamping bosses.

Optionally, each of the plurality of locking parts includes a screw threads, and the screw threads are provided on a side wall of the water inlet.

Each of the plurality of fixing pieces includes a threaded groove, the plurality of threaded grooves are provided on an inner side wall of the connector locking fastener, the threaded grooves are adapted to the thread screws in dimensions, and the plurality of threaded grooves are configured to cooperate with the thread screws and the infusion tube connector to fix the infusion tube to the water inlet hole.

The infusion tube penetrates out from the perforation hole, the infusion tube is sleeved in the insertion section, the hole entrance section is inserted into the water inlet hole, the limiting boss is propped against a hole edge of the water inlet hole, ultrasonic welding is performed on the water inlet and the infusion tube connector by using the ultrasonic line, the connector locking fastener is sleeved over the water inlet, and the plurality of threaded grooves are all screwed into the screw threads.

Another technical solution adopted in the present disclosure for solving the technical problem is as follows: respiratory equipment includes the foregoing humidifier with a glue-free chemical solvent-free dropper bottle tube connector.

Compared with the conventional art, the present disclosure provides a humidifier with a glue-free chemical solvent-free dropper bottle tube connector and respiratory equipment. A stable connection between the humidifier and an infusion tube is achieved through cooperation of an infusion tube connector, a connector locking fastener, and a locking part, which avoids a problem of loosening that may be caused by a traditional connection using glue, a curing process using the glue is abandoned, and a novel humidifier with a glue-free chemical solvent-free dropper bottle tube connector is provided, so that a safety risk caused by volatilization of volatile organic matters is eliminated, safety of a user is ensured, the use of a glue curing process is avoided, a problem caused by the volatilization of the volatile organic matters is eliminated, equipment safety is improved, air-drying curing time is not required, manufacturing time and cost for the humidifier are reduced, and production capacity of products of the humidifier is improved.

### BRIEF DESCRIPTION OF THE DRAWINGS

FIG. 1 is an exploded view of a three-dimensional structure of a humidifier with a glue-free chemical solvent-free dropper bottle tube connector according to the present disclosure.
FIG. 2 is another exploded view of a three-dimensional structure of a humidifier with a glue-free chemical solvent-free dropper bottle tube connector according to the present disclosure.
FIG. 3 is a schematic diagram of a three-dimensional structure of an infusion tube connector of a humidifier with a glue-free chemical solvent-free dropper bottle tube connector according to the present disclosure.
FIG. 4 is a front view of a humidifier with a glue-free chemical solvent-free dropper bottle tube connector according to the present disclosure.
FIG. 5 is a cross-sectional view of FIG. 4 in I-I direction.
FIG. 6 is another exploded view of a three-dimensional structure of a humidifier with a glue-free chemical solvent-free dropper bottle tube connector according to the present disclosure.
FIG. 7 is another exploded view of a three-dimensional structure of a humidifier with a glue-free chemical solvent-free dropper bottle tube connector according to the present disclosure.
FIG. 8 is another exploded view of a three-dimensional structure of a humidifier with a glue-free chemical solvent-free dropper bottle tube connector according to the present disclosure.

### Reference signs in the drawings:

10, humidifier; 11, infusion tube; 12, humidifier body; 121, upper cover end ; 122, base plate end; 13, water inlet; 131, locking part; 1311, locking boss; 1312, clamping boss; 1313, screw thread; 132, water inlet hole; 14, infusion tube connector; 141, insertion section; 142, limiting boss; 1421, ultrasonic line; 1422, inverted fastener; 143, hole entrance section; 15, connector locking fastener; 151, perforation hole; 152, fixing piece; 1521, rotary fastener; 1522, clamping groove; and 1523, threaded groove.

### DETAILED DESCRIPTION OF THE EMBODIMENTS

Embodiments of the present disclosure are described in detail below, and examples of the embodiments are shown in accompanying drawings, where the same or similar reference numerals throughout the description represent the same or similar elements or the elements having the same or similar functions. The embodiments described below with reference to the accompanying drawings are exemplary, and are only used for explaining the present disclosure and cannot be construed as a limitation to the present disclosure.

In descriptions of the present disclosure, it is to be understood that orientations or positional relationships indicated by terms "center", "longitudinal", "transverse", "upper", "lower", "front", "rear", "left", "right", "vertical", "horizontal", "top", "bottom", "inner", "outer", and the like are the orientations or positional relationships shown based on the accompanying drawings, and are merely for the convenience of describing the present disclosure and simplifying the descriptions, rather than indicating or implying that the devices or elements referred to need to have particular orientations, and constructed and operated in particular orientations. Therefore, it cannot be construed as a limitation to the present disclosure. In addition, terms "first" and "second" are used merely for the purpose of description, and cannot be construed as indicating or implying relative importance or implying a quantity of indicated technical features. Therefore, a feature limited by "first" or "second" may explicitly or implicitly include one or more of the features. In the descriptions of the present disclosure, unless otherwise specified, "a plurality of" means two or more.

In the descriptions of the present disclosure, it is to be noted that, unless otherwise specified and limited, terms "mount", "interconnect", and "connect" are to be broadly understood. For example, the terms may refer to fixed connection and may also refer to detachable connection or integration; the terms may refer to mechanical connection and may alternatively refer to electrical connection; the terms may refer to direct mutual connection, may alternatively refer to indirect connection through a medium, and may refer to communication in two components. For those of ordinary skill in the art, specific meanings of the foregoing terms in the present disclosure may be understood according to specific situations.

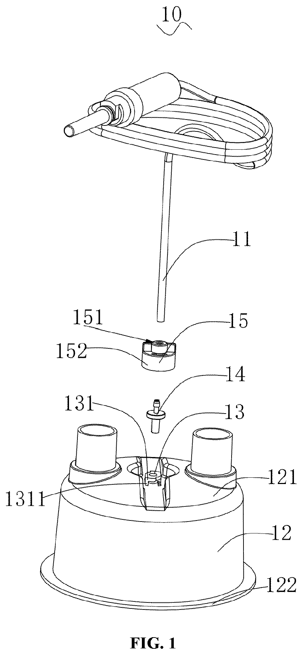

Referring to FIG. 1 to FIG. 3, a first embodiment of the present invention provides a humidifier 10 with a glue-free chemical solvent-free dropper bottle tube connector, configured for respiratory equipment. The humidifier 10 includes a humidifier body 12, a water inlet 13, an infusion tube connector 14, and a connector locking fastener 15. The humidifier body 12 has an upper cover end 121 and a base plate end 122. The water inlet 13 is fixed to the upper cover end 121. The water inlet 13 is provided with a water inlet hole 132 and a plurality of locking parts 131. The plurality of locking parts 131 are configured to connect an infusion tube 11 and the humidifier 10. The infusion tube connector 14 includes an insertion section 141, a limiting boss 142, and a hole entrance section 143. The infusion tube 11 is inserted into the infusion tube connector 14 through the insertion section 141, and the infusion tube 11 is prevented from being inserted too deeply by using the limiting boss 142. Next, the hole entrance section 143 of the infusion tube connector 14 is inserted into the water inlet hole 132 of the humidifier 10. The limiting boss 142 leans against a hole edge of the water inlet hole 132 to provide additional stability. To ensure connection stability, the locking part 131 on the side wall of the water inlet 13 cooperates with a connector locking fastener 15, and the connector locking fastener 15 is sleeved over the water inlet 13. Fixing pieces 152 on the locking fastener cooperate with the locking parts 131 by rotating or in another fixing manner, so as to firmly fix the infusion tube connector 14 to the water inlet hole 132. Meanwhile, due to a design of a perforation hole 151 in the connector locking fastener 15, the infusion tube 11 can penetrate out from this hole, so as to ensure positioning and fixing of the infusion tube 11. In this mechanical connection manner, a novel humidifier is provided, so that the use of a glue curing process is avoided, a problem about volatile organic matters is eliminated, equipment safety is improved, air-drying curing time is not required, manufacturing time and cost for the humidifier 10 are reduced, and production capacity of products of the humidifier 10 is improved.

Referring to FIG. 1 to FIG. 4, the humidifier 10 with a glue-free chemical solvent-free dropper bottle tube connector includes an infusion tube 11, a humidifier body 12, a water inlet 13, an infusion tube connector 14, and a connector locking fastener 15. The infusion tube 11 is configured to delivery liquid into the humidifier body 12. Specifically, the liquid may be distilled water, sterile water, distilled water mixed with liquid medicine, liquid medicine, or the like. The humidifier body 12 is configured to humidify gas. The humidifier body 12 has an upper cover end 121 and a base plate end 122 arranged opposite to each other. The base plate end 122 is configured to mount a heat conducting base plate. The water inlet 13 is configured to infuse the liquid guided by the infusion tube 11 into the humidifier body 12. The water inlet 13 is fixedly provided on the upper cover end 121. The water inlet 13 is provided with a water inlet hole 132. A plurality of locking parts 131 are fixedly arranged on the water inlet 13. The infusion tube connector 14 is configured to connect the infusion tube 11 to the water inlet 13. The infusion tube connector 14 has an insertion section 141, a limiting boss 142, and a hole entrance section 143. The insertion section 141, the limiting boss 142, and the hole entrance section 143 are arranged in sequence from top to bottom. The hole entrance section 143 is detachably connected to the water inlet hole 132, and the insertion section 141 is detachably connected to the infusion tube 11, so that the infusion tube 11 and the connector locking fastener 15 are easy to detach and maintain, service life of the humidifier 10 is prolonged, and convenience of the humidifier 10 is improved. The connector locking fastener 15 is provided with a perforation hole 151, and the perforation hole 151 is configured for the infusion tube 11 to penetrate out. A plurality of fixing pieces 152 are fixedly arranged on the connector locking fastener 15. The plurality of fixing pieces 152 are configured to cooperate with the locking parts 131 and the infusion tube connector 14 to fix the infusion tube 11 to the water inlet hole 132, and then a stable connection between the humidifier 10 and the infusion tube 11 is achieved through the cooperation of the infusion tube connector 14, the connector locking fastener 15, and the locking part 131, a problem of loosening that may be caused by a traditional glue connection is avoided, a curing process using glue is abandoned, a safety risk caused by volatilization of volatile organic matters is eliminated, user safety is ensured, air-drying curing time is not required, manufacturing time and cost for the humidifier 10 are reduced, and production capacity of products of the humidifier 10 is improved.

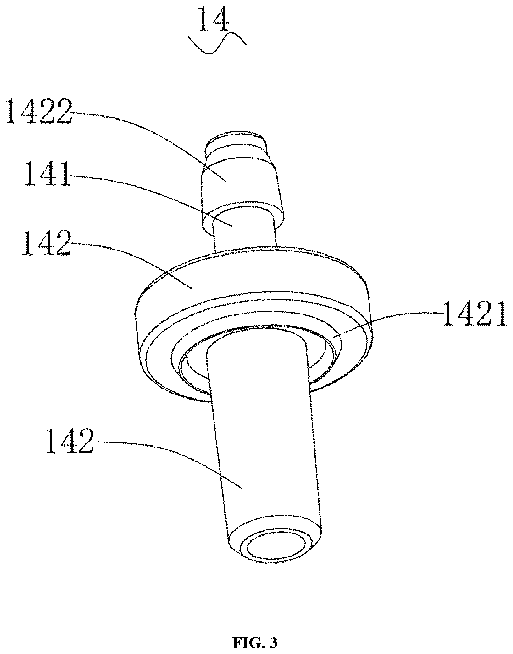

Referring to FIG. 3, the infusion tube connector 14 further includes an ultrasonic line 1421 and an inverted fastener 1422. The ultrasonic line 1421 is arranged at an end of the limiting boss 142 deviating from the insertion section 141. The ultrasonic line 1421 is arranged annularly. The ultrasonic line 1421 is configured for ultrasonic welding with the water inlet 13. The inverted fastener 1422 is configured to prevent the infusion tube 11 from separating from the infusion tube connector 14. The inverted fastener 1422 is fixedly arranged on the insertion section 141, so that a curing process using glue is abandoned, a safety risk caused by volatilization of volatile organic matters is eliminated, user safety is ensured, air-drying curing time is not required, manufacturing time and cost for the humidifier 10 are reduced, and production capacity of products of the humidifier 10 is improved.

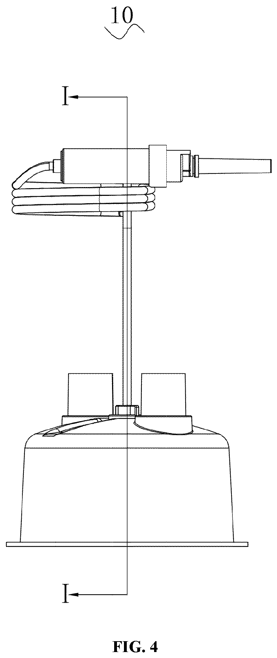

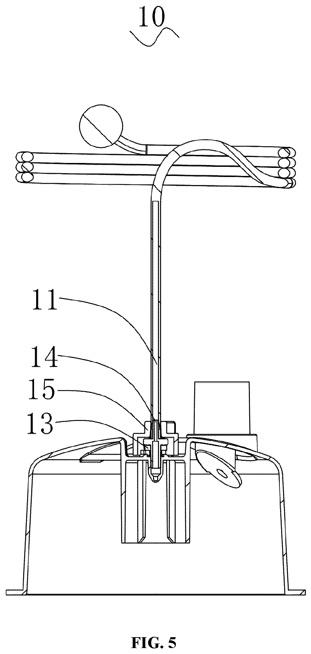

Referring to FIG. 4 and FIG. 5, in some embodiments, the inverted fastener 1422 is arranged in a cone shape. A maximum diameter of the inverted fastener 1422 is slightly greater than that of the infusion tube 11, thereby effectively preventing the infusion tube 11 from being unplugged from the inverted fastener 1422 after being stressed, and improving assembly efficiency between the infusion tube 11 and the infusion tube connector 14.

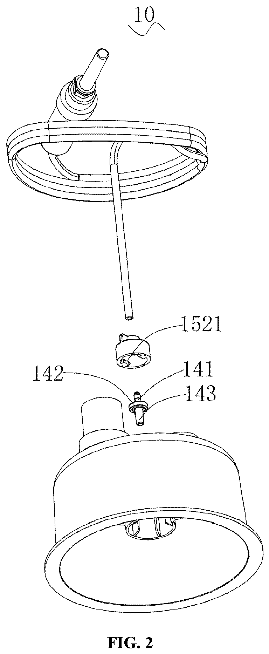

Referring to FIG. 2, in some embodiments, a cross section of the ultrasonic line 1421 along an axial direction of the infusion tube connector 14 is arranged in a V shape, a triangle, or a cone shape, which helps to concentrate and transfer energy during ultrasonic welding, and improves welding efficiency and quality, so that a connection between the infusion tube connector 14 and the water inlet 13 is more stable, a curing process using glue is abandoned, a safety risk caused by volatilization of volatile organic matters is eliminated, user safety is ensured, air-drying curing time is not required, manufacturing time and cost for the humidifier 10 are reduced, and production capacity of products of the humidifier 10 is improved.

Referring to FIG. 2, in some embodiments, the sharper the tip of the ultrasonic line 1421, the better, so that vibration energy may be concentrated at the tip of a triangle, then a uniform plastic melt flow is formed on the overall welding interface by accumulated heat, welding efficiency and quality are improved, a connection between the infusion tube connector 14 and the water inlet 13 is more stable, a curing process using glue is abandoned, a safety risk caused by volatilization of volatile organic matters is eliminated, user safety is ensured, air-drying curing time is not required, manufacturing time and cost for the humidifier 10 are reduced, and production capacity of products of the humidifier 10 is improved.

Referring to FIG. 2, in some embodiments, a welding principle of the ultrasonic line 1421 is that high-frequency friction is performed between the infusion tube connector 14 and the water inlet 13 to convert mechanical energy into thermal energy by using high-frequency vibration of a welding head of the ultrasonic line 1421, the thermal energy dissolves molecules on a welding surface between the infusion tube connector 14 and the water inlet 13 to recover activity of the molecules, and then the molecules are entangled with each other to achieve a welding purpose under assistance of an external acting force, so that air tightness and welding quality between the infusion tube connector 14 and the water inlet 13 are greatly improved, a problem of loosening that may be caused by a traditional glue connection is avoided, a curing process using glue is abandoned, a safety risk caused by volatilization of volatile organic matters is eliminated, user safety is ensured, equipment safety is improved, air-drying curing time is not required, manufacturing time and cost for the humidifier 10 are reduced, and production capacity of products of the humidifier 10 is improved.

Referring to FIG. 2, in some embodiments, a diameter of the ultrasonic line 1421 is set as 1.2 times-2 times that of the water inlet hole 132, that is, the ultrasonic line 1421 is arranged on an outer side of the water inlet hole 132, thereby optimizing a welding effect between the infusion tube connector 14 and the water inlet 13, ensuring strength and air tightness of a connection position between the infusion tube connector 14 and the water inlet 13, and improving reliability of an overall structure. The diameter of the ultrasonic line 1421 is greater than that of the water inlet hole 312, which can uniformly transfer stress during welding, stress concentration phenomena are reduced, and anti-fatigue performance of the connection position between the infusion tube connector 14 and the water inlet 13 is improved.

Referring to FIG. 1 to FIG. 5, in some embodiments, each of the plurality of locking parts 131 includes a locking boss 1311, and the locking boss 1311 is fixedly provided on a side wall of the water inlet 13, so that the infusion tube connector 14 can be quickly mounted and fixed, an assembly process is simplified, and production efficiency is improved.

Referring to FIG. 1 to FIG. 5, in some embodiments, each of the plurality of fixing pieces 152 includes a plurality of rotary fasteners 1521. The plurality of rotary fasteners 1521 are fixedly arranged on an inner side wall of the connector locking fastener 15, and the plurality of rotary fasteners 1521 are configured to cooperate with the locking boss 1311 and the infusion tube connector 14 to fix the infusion tube 11 to the water inlet hole 132. The infusion tube 11 penetrates out from the perforation hole 151, the infusion tube 11 is sleeved in the insertion section 141, the hole entrance section 143 is inserted into the water inlet hole 132, the limiting boss 142 is propped against a hole edge of the water inlet hole 132, ultrasonic welding is performed on the water inlet 13 and the infusion tube connector 14 by using the ultrasonic line 1421, the connector locking fastener 15 is sleeved over the water inlet 13, and the plurality of rotary fasteners 1521 are rotated into the locking bosses 1311 by rotating the connector locking fastener 15. Through the cooperation of the infusion tube connector 14, the locking bosses 1311, and the rotary fasteners 1521, a stable connection between the humidifier 10 and the infusion tube 11 is achieved, a problem of loosening that may be caused by a traditional glue connection is avoided, a curing process using glue is abandoned, a safety risk caused by volatilization of volatile organic matters is eliminated, user safety is ensured, equipment safety is improved, air-drying curing time is not required, manufacturing time and cost for the humidifier 10 are reduced, and production capacity of products of the humidifier 10 is improved.

In some embodiments, the locking boss 1311 includes a horizontal boss, a vertical boss, and a clamping boss. The horizontal boss and the vertical boss are provided perpendicularly to form a locking space. The horizontal boss and the vertical boss are integrally arranged. The limiting boss 142 is arranged on the upper cover end 121. The limiting boss 142 is configured to prevent the plurality of rotary fasteners 1521 from sliding out from the locking space, thereby effectively preventing the infusion tube 11 from falling during use, and improving safety and reliability of a connection between the infusion tube connector 14 and the water inlet 13.

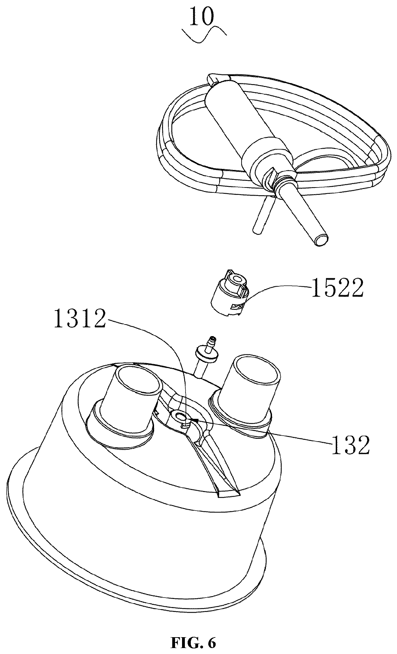

Referring to FIG. 6, in some embodiments, each of the plurality of locking parts 131 includes a clamping boss 1312, and the clamping boss 1312 is provided on a side wall of the water inlet 13, so that the infusion tube connector 14 can be quickly mounted and fixed, an assembly process is simplified, and production efficiency is improved.

Referring to FIG. 6, in some embodiments, an end face of the clamping boss 1312 is flush with an end face of the water inlet 13, so that a clamping difficulty between the connector locking fastener 15 and the water inlet 13 is reduced, the infusion tube connector 14 can be quickly mounted and fixed, an assembly process is simplified, and production efficiency is improved.

Referring to FIG. 6, in some embodiments, each of the plurality of fixing pieces 152 includes a clamping groove 1522. The plurality of clamping grooves 1522 are provided in a side wall of the connector locking fastener 15. The clamping bosses 1312 are adapted to the clamping grooves 1522 in dimensions. The plurality of clamping grooves 1522 are configured to cooperate with the clamping boss 1312 and the infusion tube connector 14 to fix the infusion tube 11 to the water inlet hole 132. The infusion tube 11 penetrates out from the perforation hole 151. The infusion tube 11 is sleeved in the insertion section 141. The hole entrance section 143 is inserted into the water inlet hole 132. The limiting boss 142 is propped against a hole edge of the water inlet hole 132. Ultrasonic welding is performed on the water inlet 13 and the infusion tube connector 14 by using the ultrasonic line 1421. The connector locking fastener 15 is sleeved over the water inlet 13. The plurality of clamping grooves 1522 are all buckled in the clamping bosses 1312, so that air tightness and welding quality between the infusion tube connector 14 and the water inlet 13 are greatly improved, a problem of loosening that may be caused by a traditional glue connection is avoided, a curing process using glue is abandoned, a safety risk caused by volatilization of volatile organic matters is eliminated, user safety is ensured, safety of the humidifier 10 is improved, air-drying curing time is not required, manufacturing time and cost for the humidifier 10 are reduced, and production capacity of products of the humidifier 10 is improved.

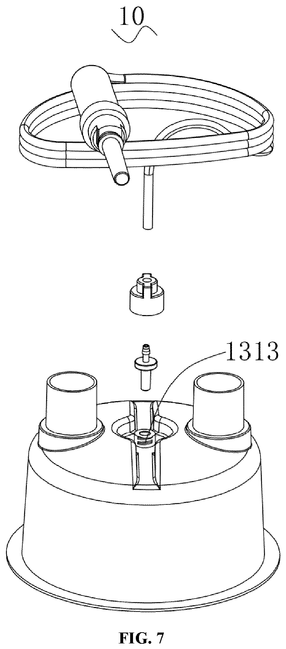

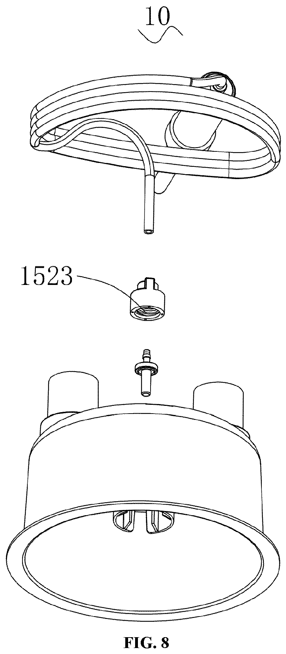

Referring to FIG. 7 and FIG. 8, in some embodiments, each of the plurality of locking parts 131 includes screw threads 1313. The screw threads 1313 are provided on a side wall of the water inlet 13. Each of the plurality of fixing pieces 152 includes a threaded groove 1523. The plurality of threaded grooves 1523 are provided on an inner side wall of the connector locking fastener 15. The threaded grooves 1523 are adapted to the screw threads 1313 in dimensions. The plurality of threaded grooves 1523 are configured to cooperate with the screw threads 1313 and the infusion tube connector 14 to fix the infusion tube 11 to the water inlet hole 132, so that air tightness between the water inlet 13 and the connector locking fastener 15 is improved, liquid leakage is prevented, and safety and reliability of the humidifier 10 are improved. The infusion tube 11 penetrates out from the perforation hole 151, the infusion tube 11 is sleeved in the insertion section 141, the hole entrance section 143 is inserted into the water inlet hole 132, the limiting boss 142 is propped against a hole edge of the water inlet hole 132, ultrasonic welding is performed on the water inlet 13 and the infusion tube connector 14 by using the ultrasonic line 1421, the connector locking fastener 15 is sleeved over the water inlet 13, and the plurality of threaded grooves 1523 are all screwed into the screw threads 1313, so that air tightness and welding quality between the infusion tube connector 14 and the water inlet 13 are greatly improved, a problem of loosening that may be caused by a traditional glue connection is avoided, a curing process using glue is abandoned, a safety risk caused by volatilization of volatile organic matters is eliminated, user safety is ensured, air-drying curing time is not required, manufacturing time and cost for the humidifier 10 are reduced, and production capacity of products of the humidifier 10 is improved.

In some embodiments, "glue-free chemical solvent-free" in the humidifier 10 with a glue-free chemical solvent-free dropper bottle tube connector in the present disclosure refers to that glue or other chemical solvents are not used during an assembly process of the humidifier body 12 and the infusion tube 11, so that a problem of loosening that may be caused by a traditional glue connection is avoided, a curing process using glue is abandoned, a safety risk caused by volatilization of volatile organic matters is eliminated, user safety is ensured, air-drying curing time is not required, manufacturing time and cost for the respiratory equipment are reduced, and production capacity of the respiratory equipment is improved.

A second embodiment of the present invention provides respiratory equipment, including the humidifier with a glue-free chemical solvent-free dropper bottle tube connector as described above, so that air tightness and welding quality between the infusion tube connector and the water inlet are greatly improved, a problem of loosening that may be caused by a traditional glue connection is avoided, a curing process using glue is abandoned, a safety risk caused by volatilization of volatile organic matters is eliminated, user safety is ensured, moreover, air-drying curing time is not required, manufacturing time and cost for the respiratory equipment are reduced, and production capacity of the respiratory equipment is improved.

In conclusion, the present disclosure provides a humidifier with a glue-free chemical solvent-free dropper bottle tube connector and respiratory equipment. The humidifier includes an infusion tube; a humidifier body, where the humidifier body has an upper cover end and a base plate end arranged opposite to each other; a water inlet, where the water inlet is fixedly provided on the upper cover end, the water inlet is provided with a water inlet hole, and a plurality of locking parts are fixedly arranged on the water inlet; an infusion tube connector, where the infusion tube connector has an insertion section, a limiting boss, and a hole entrance section arranged in sequence from top to bottom, the hole entrance section is detachably connected to the water inlet hole, and the insertion section is detachably connected to the infusion tube; and a connector locking fastener, where the connector locking fastener is provided with a perforation hole, a plurality of fixing pieces are fixedly arranged on the connector locking fastener, and the plurality of fixing pieces are configured to cooperate with the locking parts and the infusion tube connector to fix the infusion tube to the water inlet hole. A novel humidifier with a glue-free chemical solvent-free dropper bottle tube connector is provided, so that air tightness and welding quality between the infusion tube connector and the water inlet are greatly improved, a problem of loosening that may be caused by a traditional glue connection is avoided, a curing process using glue is abandoned, a safety risk caused by volatilization of volatile organic matters is eliminated, user safety is ensured, air-drying curing time is not required, manufacturing time and cost for the humidifier are reduced, and production capacity of products of the humidifier is improved.

It is to be understood that applications of the present disclosure are not limited to the foregoing examples but limited to the scope of the claims attached to the present disclosure.

## Claims

1. A humidifier (10) with a glue-free chemical solvent-free dropper bottle tube connector, configured for respiratory equipment, comprising:
an infusion tube (11);
a humidifier body (12), wherein the humidifier body (12) has an upper cover end (121) and a base plate end (122) arranged opposite to each other;
a water inlet (13), wherein the water inlet (13) is fixedly provided on the upper cover end (121), the water inlet (13) is provided with a water inlet hole (132), and a plurality of locking parts (131) are fixedly arranged on the water inlet (13);
an infusion tube connector (14), wherein the infusion tube connector (14) has an insertion section (141), a limiting boss (142), and a hole entrance section (143) arranged in sequence from top to bottom, the hole entrance section (143) is detachably connected to the water inlet hole (132), and the insertion section (141) is detachably connected to the infusion tube (11); and
a connector locking fastener (15), wherein the connector locking fastener (15) is provided with a perforation hole (151), a plurality of fixing pieces (152) are fixedly arranged on the connector locking fastener (15), and the plurality of fixing pieces (152) are configured to cooperate with the locking parts (131) and the infusion tube connector (14) to fix the infusion tube (11) to the water inlet hole (132), wherein the infusion tube connector (14) further comprises:
an ultrasonic line (1421), wherein the ultrasonic line (1421) is annularly arranged at an end of the limiting boss (142) deviating from the insertion section (141), and the ultrasonic line (1421) is configured for ultrasonic welding with the water inlet (13); and
an inverted fastener (1422), wherein the inverted fastener (1422) is fixedly arranged on the insertion section (141).

2. The humidifier (10) according to claim 1, wherein a cross section of the ultrasonic line (1421) along an axial direction of the infusion tube connector (14) is arranged in a V shape, a triangle, or a cone shape.

3. The humidifier (10) according to claim 1, wherein a diameter of the ultrasonic line (1421) is set as 1.2 times-2 times that of the water inlet hole (132).

4. The humidifier (10) according to claim 1, wherein each of the plurality of locking parts (131) comprises a locking boss (1311), and the locking boss (1311) is fixedly provided on a side wall of the water inlet (13).

5. A method of fixing the infusion tube (11) to the water inlet hole (132) of the humidifier (10) of claim 4, wherein each of the plurality of fixing pieces (152) comprises a plurality of rotary fasteners (1521), the plurality of rotary fasteners (1521) are fixedly arranged on an inner side wall of the connector locking fastener (15), and the plurality of rotary fasteners (1521) are configured to cooperate with the locking boss (1311) and the infusion tube connector (14) to fix the infusion tube (11) to the water inlet hole (132), the method comprising:
causing the infusion tube (11) to penetrate out from the perforation hole (151);
sleeving the infusion tube (11) in the insertion section (141);
inserting the hole entrance section (143) into the water inlet hole (132):
propping the limiting boss (142) against a hole edge of the water inlet hole (132):
performing ultrasonic welding on the water inlet (13) and the infusion tube connector (14) by using the ultrasonic line (1421);
sleeving the connector locking fastener (15) over the water inlet (13), and
rotating the plurality of rotary fasteners (1521) into the locking bosses (1311) by rotating the connector locking fastener (15).

6. The humidifier (10) according to claim 1, wherein each of the plurality of locking parts (131) comprises a clamping boss (1312), and the clamping boss (1312) is provided on a side wall of the water inlet (13).

7. A method of fixing the infusion tube (11) to the water inlet hole (132) of the humidifier (10) of claim 6, wherein each of the plurality of fixing pieces (152) comprises a clamping groove (1522), the plurality of clamping grooves (1522) are provided in a side wall of the connector locking fastener (15), the clamping bosses (1312) are adapted to the clamping grooves (1522) in dimensions, and the plurality of clamping grooves (1522) are configured to cooperate with the clamping bosses (1312) and the infusion tube connector (14) to fix the infusion tube (11) to the water inlet hole (132), the method comprising:
causing the infusion tube (11) to penetrate out from the perforation hole (151);
sleeving the infusion tube (11) in the insertion section (141);
inserting the hole entrance section (143) into the water inlet hole (132):
propping the limiting boss (142) against a hole edge of the water inlet hole (132):
performing ultrasonic welding on the water inlet (13) and the infusion tube connector (14) by using the ultrasonic line (1421);
sleeving the connector locking fastener (15) over the water inlet (13), and
buckling all of the plurality of clamping grooves (1522) in the clamping bosses (1312).

8. A method of fixing the infusion tube (11) to the water inlet hole (132) of the humidifier (10) of claim 1, wherein each of the plurality of locking parts (131) comprises screw threads, and the screw threads are provided on a side wall of the water inlet (13); each of the plurality of fixing pieces (152) comprises a threaded groove (1523), the plurality of threaded grooves (1523) are provided on an inner side wall of the connector locking fastener (15), the threaded grooves (1523) are adapted to the screw threads in dimensions, and the plurality of threaded grooves (1523) are configured to cooperate with the screw threads and the infusion tube connector (14) to fix the infusion tube (11) to the water inlet hole (132), the method comprising:
causing the infusion tube (11) to penetrate out from the perforation hole (151);
sleeving the infusion tube (11) in the insertion section (141);
inserting the hole entrance section (143) into the water inlet hole (132):
propping the limiting boss (142) against a hole edge of the water inlet hole (132):
performing ultrasonic welding on the water inlet (13) and the infusion tube connector (14) by using the ultrasonic line (1421);
sleeving the connector locking fastener (15) over the water inlet (13); and
screwing the plurality of threaded grooves (1523) into the screw threads.

9. Respiratory equipment, comprising the humidifier (10) according to any one of claims 1 to 4 and 6.

## Patentansprüche

1. Befeuchter (10) mit einem kleber- und chemikalienlösungsmittelfreien Tropfflaschen-Rohrverbinder, der für Atemgeräte eingerichtet ist, umfassend:
ein Infusionsrohr (11);
einen Befeuchterkörper (12), wobei der Befeuchterkörper (12) ein Oberdeckelende (121) und ein Bodenplattenende (122) aufweist, die einander gegenüberliegend angeordnet sind;
einen Wassereinlass (13), wobei der Wassereinlass (13) fest am Oberdeckelende (121) vorgesehen ist, wobei der Wassereinlass (13) mit einer Wassereinlassbohrung (132) versehen ist, wobei am Wassereinlass (13) mehrere Verriegelungsteile (131) fest angeordnet sind;
einen Infusionsrohrverbinder (14), wobei der Infusionsrohrverbinder (14) von oben nach unten nacheinander einen Einsteckabschnitt (141), einen Begrenzungsansatz (142) und einen Bohrungseintrittsabschnitt (143) aufweist, wobei der Bohrungseintrittsabschnitt (143) lösbar mit der Wassereinlassbohrung (132) verbunden ist, wobei der Einsteckabschnitt (141) lösbar mit dem Infusionsrohr (11) verbunden ist; und
einen Verbinder-Riegelverschluss (15), wobei der Verbinder-Riegelverschluss (15) mit einer Durchtrittsbohrung (151) versehen ist, wobei auf dem Verbinder-Riegelverschluss (15) mehrere Befestigungselemente (152) fest angeordnet sind, wobei die mehreren Befestigungselemente (152) dazu eingerichtet sind, mit den Verriegelungsteilen (131) und dem Infusionsrohrverbinder (14) zusammenzuwirken, um das Infusionsrohr (11) an der Wassereinlassbohrung (132) zu befestigen, wobei der Infusionsrohrverbinder (14) ferner Folgendes umfasst:
eine Ultraschallnaht (1421), wobei die Ultraschallnaht (1421) ringförmig an einem vom Einsteckabschnitt (141) abgewandten Ende des Begrenzungsansatzes (142) angeordnet ist, wobei die Ultraschallnaht (1421) für ein Ultraschallschweißen mit dem Wassereinlass (13) eingerichtet ist; und
einen invertierten Verschluss (1422), wobei der invertierte Verschluss (1422) fest auf dem Einsteckabschnitt (141) angeordnet ist.

2. Befeuchter (10) nach Anspruch 1, wobei ein Querschnitt der Ultraschallnaht (1421) entlang einer Axialrichtung des Infusionsrohrverbinders (14) V-förmig, dreieckig oder konisch ausgebildet ist.

3. Befeuchter (10) nach Anspruch 1, wobei ein Durchmesser der Ultraschallnaht (1421) auf das 1,2-fache bis 2-fache des Durchmessers der Wassereinlassbohrung (132) eingestellt ist.

4. Befeuchter (10) nach Anspruch 1, wobei jedes der mehreren Verriegelungsteile (131) einen Verriegelungsansatz (1311) umfasst, wobei der Verriegelungsansatz (1311) fest auf einer Seitenwand des Wassereinlasses (13) angeordnet ist.

5. Verfahren zum Befestigen des Infusionsrohrs (11) an der Wassereinlassbohrung (132) des Befeuchters (10) nach Anspruch 4, wobei jedes der mehreren Befestigungselemente (152) mehrere Drehverschlüsse (1521) umfasst, wobei die mehreren Drehverschlüsse (1521) fest auf einer Innenseitenwand des Verbinder-Riegelverschlusses (15) angeordnet sind, wobei die mehreren Drehverschlüsse (1521) dazu eingerichtet sind, mit dem Verriegelungsansatz (1311) und dem Infusionsrohrverbinder (14) zusammenzuwirken, um das Infusionsrohr (11) an der Wassereinlassbohrung (132) zu befestigen, wobei das Verfahren Folgendes umfasst:
Veranlassen, dass das Infusionsrohr (11) aus der Durchtrittsbohrung (151) durchtritt;
Aufstecken des Infusionsrohrs (11) auf den Einsteckabschnitt (141);
Einführen des Bohrungseintrittsabschnitts (143) in die Wassereinlassbohrung (132);
Anlegen des Begrenzungsansatzes (142) an einen Bohrungsrand der Wassereinlassbohrung (132);
Durchführen eines Ultraschallschweißens des Wassereinlasses (13) und des Infusionsrohrverbinders (14) unter Verwendung der Ultraschallnaht (1421);
Aufstecken des Verbinder-Riegelverschlusses (15) auf den Wassereinlass (13); und
Eindrehen der mehreren Drehverschlüsse (1521) in die Verriegelungsansätze (1311) durch Drehen des Verbinder-Riegelverschlusses (15).

6. Befeuchter (10) nach Anspruch 1, wobei jedes der mehreren Verriegelungsteile (131) einen Klemmansatz (1312) umfasst, wobei der Klemmansatz (1312) auf einer Seitenwand des Wassereinlasses (13) angeordnet ist.

7. Verfahren zum Befestigen des Infusionsrohrs (11) an der Wassereinlassbohrung (132) des Befeuchters (10) nach Anspruch 6, wobei jedes der mehreren Befestigungselemente (152) eine Klemmnut (1522) umfasst, wobei die mehreren Klemmnuten (1522) in einer Seitenwand des Verbinder-Riegelverschlusses (15) angeordnet sind, wobei die Klemmansätze (1312) in ihren Abmessungen an die Klemmnuten (1522) angepasst sind, wobei die mehreren Klemmnuten (1522) dazu eingerichtet sind, mit den Klemmansätzen (1312) und dem Infusionsrohrverbinder (14) zusammenzuwirken, um das Infusionsrohr (11) an der Wassereinlassbohrung (132) zu befestigen, wobei das Verfahren Folgendes umfasst:
Veranlassen, dass das Infusionsrohr (11) aus der Durchtrittsbohrung (151) durchtritt;
Aufstecken des Infusionsrohrs (11) auf den Einsteckabschnitt (141);
Einführen des Bohrungseintrittsabschnitts (143) in die Wassereinlassbohrung (132);
Anlegen des Begrenzungsansatzes (142) an einen Bohrungsrand der Wassereinlassbohrung (132);
Durchführen eines Ultraschallschweißens des Wassereinlasses (13) und des Infusionsrohrverbinders (14) unter Verwendung der Ultraschallnaht (1421);
Aufstecken des Verbinder-Riegelverschlusses (15) auf den Wassereinlass (13); und
Einschnappen aller der mehreren Klemmnuten (1522) in die Klemmansätze (1312).

8. Verfahren zum Befestigen des Infusionsrohrs (11) an der Wassereinlassbohrung (132) des Befeuchters (10) nach Anspruch 1, wobei jedes der mehreren Verriegelungsteile (131) Schraubgewinde umfasst, wobei die Schraubgewinde auf einer Seitenwand des Wassereinlasses (13) vorgesehen sind, wobei jedes der mehreren Befestigungselemente (152) eine Gewindenut (1523) umfasst, wobei die mehreren Gewindenuten (1523) auf einer Innenseitenwand des Verbinder-Riegelverschlusses (15) vorgesehen sind, wobei die Gewindenuten (1523) in ihren Abmessungen an die Schraubgewinde angepasst sind, wobei die mehreren Gewindenuten (1523) dazu eingerichtet sind, mit den Schraubgewinden und dem Infusionsrohrverbinder (14) zusammenzuwirken, um das Infusionsrohr (11) an der Wassereinlassbohrung (132) zu befestigen, wobei das Verfahren Folgendes umfasst:
Veranlassen, dass das Infusionsrohr (11) aus der Durchtrittsbohrung (151) durchtritt;
Aufstecken des Infusionsrohrs (11) auf den Einsteckabschnitt (141);
Einführen des Bohrungseintrittsabschnitts (143) in die Wassereinlassbohrung (132);
Anlegen des Begrenzungsansatzes (142) an einen Bohrungsrand der Wassereinlassbohrung (132);
Durchführen eines Ultraschallschweißens des Wassereinlasses (13) und des Infusionsrohrverbinders (14) unter Verwendung der Ultraschallnaht (1421);
Aufstecken des Verbinder-Riegelverschlusses (15) auf den Wassereinlass (13); und
Einschrauben der mehreren Gewindenuten (1523) in die Schraubgewinde.

9. Atemgerät, umfassend einen Befeuchter (10) nach einem der Ansprüche 1 bis 4 und 6.

## Revendications

1. Humidificateur (10) pourvu d'un raccord de tube pour flacon compte-gouttes sans colle et sans solvant chimique, conçu pour un équipement respiratoire, comprenant :
un tube de perfusion (11) ;
un corps d'humidificateur (12), dans lequel le corps d'humidificateur (12) a une extrémité de couvercle supérieur (121) et une extrémité de plaque de base (122) disposées à l'opposé l'une de l'autre ;
une entrée d'eau (13), dans lequel l'entrée d'eau (13) est prévue de manière fixe sur l'extrémité de couvercle supérieur (121), l'entrée d'eau (13) est pourvue d'un orifice d'entrée d'eau (132), et une pluralité d'éléments de verrouillage (131) sont disposés de manière fixe sur l'entrée d'eau (13) ;
un raccord de tube de perfusion (14), dans lequel le raccord de tube de perfusion (14) a une section d'insertion (141), un bossage de limitation (142) et une section d'entrée d'orifice (143) disposées successivement de haut en bas, la section d'entrée d'orifice (143) étant raccordée de manière amovible à l'orifice d'entrée d'eau (132), et la section d'insertion (141) est raccordée de manière amovible au tube de perfusion (11) ; et
une fixation de verrouillage de raccord (15), dans lequel la fixation de verrouillage de raccord (15) est pourvue d'un trou de perforation (151), une pluralité de pièces de fixation (152) sont disposés de manière fixe sur la fixation de verrouillage de raccord (15), et la pluralité de pièces de fixation (152) sont conçus pour coopérer avec les éléments de verrouillage (131) et le raccord de tube de perfusion (14) afin de fixer le tube de perfusion (11) à l'orifice d'entrée d'eau (132), dans lequel le raccord de tube de perfusion (14) comprend en outre :
une ligne ultrasonique (1421), dans lequel la ligne ultrasonique (1421) est disposée de manière annulaire au niveau d'une extrémité du bossage de limitation (142) s'écartant de la section d'insertion (141), et la ligne ultrasonique (1421) est conçue pour un soudage par ultrasons avec l'entrée d'eau (13) ; et
une fixation inversée (1422), dans lequel la fixation inversée (1422) est disposée de manière fixe sur la section d'insertion (141).

2. Humidificateur (10) selon la revendication 1, dans lequel une section transversale de la ligne ultrasonique (1421) le long d'une direction axiale du raccord de tube de perfusion (14) est disposée en forme de V, de triangle ou de cône.

3. Humidificateur (10) selon la revendication 1, dans lequel un diamètre de la ligne ultrasonique (1421) est défini comme étant compris entre 1,2 fois et 2 fois celui de l'orifice d'entrée d'eau (132).

4. Humidificateur (10) selon la revendication 1, dans lequel chacun de la pluralité d'éléments de verrouillage (131) comprend un bossage de verrouillage (1311), et le bossage de verrouillage (1311) est prévu de manière fixe sur une paroi latérale de l'entrée d'eau (13).

5. Procédé destiné à fixer le tube de perfusion (11) à l'orifice d'entrée d'eau (132) de l'humidificateur (10) selon la revendication 4, dans lequel chacune de la pluralité de pièces de fixation (152) comprend une pluralité de fixations rotatives (1521), la pluralité de fixations rotatives (1521) sont disposées de manière fixe sur une paroi latérale interne de la fixation de verrouillage de raccord (15), et la pluralité de fixations rotatives (1521) sont conçues pour coopérer avec le bossage de verrouillage (1311) et le raccord de tube de perfusion (14) afin de fixer le tube de perfusion (11) à l'orifice d'entrée d'eau (132), le procédé comprenant :
le fait d'amener le tube de perfusion (11) à sortir hors du trou de perforation (151) ;
le gainage du tube de perfusion (11) dans la section d'insertion (141) ;
l'insertion de la section d'entrée d'orifice (143) dans l'orifice d'entrée d'eau (132) ;
la mise en appui du bossage de limitation (142) contre un bord d'orifice de l'orifice d'entrée d'eau (132) ;
la réalisation d'un soudage par ultrasons sur l'entrée d'eau (13) et le raccord de tube de perfusion (14) à l'aide de la ligne ultrasonique (1421) ;
le gainage de la fixation de verrouillage de raccord (15) sur l'entrée d'eau (13), et
la rotation de la pluralité de fixations rotatives (1521) dans les bossages de verrouillage (1311) en faisant tourner la fixation de verrouillage de raccord (15).

6. Humidificateur (10) selon la revendication 1, dans lequel chacun de la pluralité d'éléments de verrouillage (131) comprend un bossage de serrage (1312), et le bossage de serrage (1312) est prévu sur une paroi latérale de l'entrée d'eau (13).

7. Procédé destiné à fixer le tube de perfusion (11) à l'orifice d'entrée d'eau (132) de l'humidificateur (10) selon la revendication 6, dans lequel chacune de la pluralité de pièces de fixation (152) comprend une rainure de serrage (1522), la pluralité de rainures de serrage (1522) sont prévues dans une paroi latérale de la fixation de verrouillage de raccord (15), les bossage de serrage (1312) sont adaptés aux rainures de serrage (1522) en termes de dimensions, et la pluralité de rainures de serrage (1522) sont conçues pour coopérer avec les bossage de serrage (1312) et le raccord de tube de perfusion (14) afin de fixer le tube de perfusion (11) à l'orifice d'entrée d'eau (132), le procédé comprenant :
le fait d'amener le tube de perfusion (11) à sortir hors du trou de perforation (151) ;
le gainage du tube de perfusion (11) dans la section d'insertion (141) ;
l'insertion de la section d'entrée d'orifice (143) dans l'orifice d'entrée d'eau (132) ;
la mise en appui du bossage de limitation (142) contre un bord d'orifice de l'orifice d'entrée d'eau (132) ;
la réalisation d'un soudage par ultrasons sur l'entrée d'eau (13) et le raccord de tube de perfusion (14) à l'aide de la ligne ultrasonique (1421) ;
le gainage de la fixation de verrouillage de raccord (15) sur l'entrée d'eau (13), et
le flambage de la totalité de la pluralité de rainures de serrage (1522) dans les bossage de serrage (1312).

8. Procédé destiné à fixer le tube de perfusion (11) à l'orifice d'entrée d'eau (132) de l'humidificateur (10) selon la revendication 1, dans lequel chacun de la pluralité d'éléments de verrouillage (131) comprend des filets de vis, et les filets de vis sont prévus sur une paroi latérale de l'entrée d'eau (13) ; chacune de la pluralité de pièces de fixation (152) comprend une rainure filetée (1523), la pluralité de rainures filetées (1523) sont prévues sur une paroi latérale interne de la fixation de verrouillage de raccord (15), les rainures filetées (1523) sont adaptées aux filets de vis en termes de dimensions, et la pluralité de rainures filetées (1523) sont conçues pour coopérer avec les filets de vis et le raccord de tube de perfusion (14) afin de fixer le tube de perfusion (11) à l'orifice d'entrée d'eau (132), le procédé comprenant :
le fait d'amener le tube de perfusion (11) à sortir hors du trou de perforation (151) ;
le gainage du tube de perfusion (11) dans la section d'insertion (141) ;
l'insertion de la section d'entrée d'orifice (143) dans l'orifice d'entrée d'eau (132) ;
la mise en appui du bossage de limitation (142) contre un bord d'orifice de l'orifice d'entrée d'eau (132) ;
la réalisation d'un soudage par ultrasons sur l'entrée d'eau (13) et le raccord de tube de perfusion (14) à l'aide de la ligne ultrasonique (1421) ;
le gainage de la fixation de verrouillage de raccord (15) sur l'entrée d'eau (13) ; et
le vissage de la pluralité de rainures filetées (1523) dans les filets de vis.

9. Équipement respiratoire, comprenant l'humidificateur (10) selon l'une quelconque des revendications 1 à 4 et 6.
